# EUROPEAN PATENT APPLICATION

(11) **EP 3 922 263 A1**
(43) Date of publication of application: **15.12.2021**
(21) Application number: 21763207.4
(22) Date of filing: 02.02.2021
(51) Int. Cl.: A61K 39/215, A61P 31/14, A61P 11/00

(54) **AD7 VECTOR VACCINE FOR PREVENTING SARS-COV-2 INFECTION**

(30) Priority: 16.03.2020 CN 202010182122; 15.04.2020 CN 202010293658; 27.09.2020 CN 202011031062
(71) Applicant: Guangzhou N Biomed Ltd., Guangzhou, Guangdong 510663 (CN)
(72) Inventor: CHEN, Ling, Guangdong 510663 (CN); GUAN, Suhua, Guangdong 510663 (CN); YANG, Chenchen, Guangdong 510663 (CN); WANG, Qian, Guangdong 510663 (CN)
(74) Representative: Gassner, Birgitta
(86) International application number: PCT/CN2021/074837
(87) International publication number: WO 2021/184987

(57) **Abstract**

Disclosed is an Ad7-vectored vaccine for preventing SARS-CoV-2 infection, which is an adenovirus vectored vaccine, comprising an Ad7 vector loaded with a nucleic acid sequence shown in SEQ ID NO: 1. Some embodiments of the disclosure have good safety and are convenient to use. Experiments have shown that the vaccine is capable of producing more S protein in human cells, which is expected to be developed as a vaccine for preventing SARS-CoV-2 infection. Some embodiments of the disclosure may be used in combination with other vaccines, and may also be used as therapeutic vaccines for COVID-19. When a patient is vaccinated with the Ad7-vectored vaccine of the present disclosure at the initial stage of infection, the vaccine can quickly induces immune response in the human body, thereby achieving a therapeutic effect.

SEQUENCE LISTING

<110>  GUANGZHOU N BIOMED LTD.

<120>  AD7-VECTORED VACCINE FOR PREVENTING SARS-COV-2 INFECTION

<130>  Ad7

<160>  7

<170>  PatentIn version 3.5

<210>  1
<211>  3822
<212>  DNA
<213> Artificial Sequence

<400>  1
atgttcgtgt ttctggtgct gctgcctctg gtgagctccc agtgcgtgaa cctgaccaca      60

aggacccagc tgccacctgc ctataccaat agcttcacac ggggcgtgta ctatcccgac     120

aaggtgttta gatctagcgt gctgcactcc acccaggatc tgtttctgcc tttcttttct     180

aacgtgacat ggttccacgc catccacgtg tccggcacca atggcacaaa gcggttcgac     240

aatccagtgc tgccctttaa cgatggcgtg tacttcgcct ccaccgagaa gtctaacatc     300

atcagaggct ggatctttgg caccacactg gacagcaaga cccagtccct gctgatcgtg     360

aacaatgcca caaacgtggt catcaaggtg tgcgagttcc agttttgtaa tgatcccttc     420

ctgggcgtgt actatcacaa gaacaataag tcttggatgg agagcgagtt tagggtgtat     480

tcctctgcca acaattgcac ctttgagtac gtgagccagc ctttcctgat ggacctggag     540

ggcaagcagg gcaatttcaa gaacctgagg gagttcgtgt ttaagaatat cgatggctac     600

ttcaagatct actccaagca cacaccaatc aacctggtgc gcgacctgcc acagggcttc     660

tctgccctgg agccactggt ggatctgccc atcggcatca acatcacccg gtttcagaca     720

ctgctggccc tgcacagaag ctacctgacc ccaggcgaca gctcctctgg atggacagca     780

ggagctgccg cctactatgt gggctatctg cagccccgca ccttcctgct gaagtacaac     840

gagaatggca ccatcacaga cgcagtggat tgcgccctgg accccctgtc tgagaccaag     900

tgtacactga agagctttac agtggagaag ggcatctacc agaccagcaa cttcagggtg     960

cagccaacag agtccatcgt gcgctttccc aatatcacca acctgtgccc ttttggcgag    1020

gtgttcaatg ccacacgctt cgccagcgtg tacgcctgga ataggaagcg catctccaac    1080

tgcgtggccg actattctgt gctgtacaac agcgcctcct tctctacctt taagtgttat    1140

ggcgtgagcc ccaccaagct gaatgatctg tgctttacaa acgtgtacgc cgattccttc    1200

gtgatcaggg gcgacgaggt gcgccagatc gcaccaggac agaccggcaa gatcgcagac    1260

tacaattata agctgcctga cgatttcaca ggctgcgtga tcgcctggaa ctctaacaat    1320

ctggatagca aagtgggcgg caactacaat tatctgtacc ggctgtttag aaagtctaat    1380

ctgaagccat tcgagcggga catctccacc gagatctacc aggccggctc tacaccctgc 1440

aatggcgtgg agggctttaa ctgttatttc cctctgcagt cctacggctt ccagccaacc 1500

aacggcgtgg gctatcagcc ctacagagtg gtggtgctgt cttttgagct gctgcacgca 1560

cctgcaaccg tgtgcggccc aaagaagagc acaaatctgg tgaagaacaa gtgcgtgaac 1620

ttcaacttca acggactgac cggcacaggc gtgctgaccg agagcaacaa gaagttcctg 1680

ccatttcagc agttcggcag ggacatcgca gataccacag acgccgtgcg cgaccctcag 1740

accctggaga tcctggacat cacaccatgt tccttcggcg gcgtgtctgt gatcacccca 1800

ggcaccaata catccaacca ggtggccgtg ctgtatcagg acgtgaattg cacagaggtg 1860

cccgtggcaa tccacgcaga tcagctgacc cctacatggc gggtgtactc taccggcagc 1920

aacgtgttcc agacaagagc cggatgcctg atcggagcag agcacgtgaa caatagctat 1980

gagtgcgaca tccctatcgg cgccggcatc tgtgcctcct accagaccca gacaaactcc 2040

ccaaggagag cccggtctgt ggccagccag tccatcatcg cctataccat gagcctgggc 2100

gccgagaaca gcgtggccta ctccaacaat tctatcgcca tccctaccaa cttcacaatc 2160

agcgtgacca cagagatcct gccagtgagc atgaccaaga catccgtgga ctgcaccatg 2220

tatatctgtg gcgattccac agagtgttct aacctgctgc tgcagtacgg ctccttttgc 2280

acccagctga atagagccct gacaggcatc gccgtggagc aggacaagaa cacccaggag 2340

gtgttcgccc aggtgaagca gatctacaag acaccaccca tcaaggactt tggcggcttc 2400

aacttcagcc agatcctgcc cgatcctagc aagccatcca agcggtcttt tatcgaggac 2460

ctgctgttca acaaggtgac cctggccgat gccggcttca tcaagcagta tggcgattgt 2520

ctgggcgaca tcgccgccag agacctgatc tgcgcccaga agtttaatgg cctgaccgtg 2580

ctgcctccac tgctgacaga tgagatgatc gcacagtaca cctctgccct gctggccggc 2640

accatcacaa gcggatggac attcggcgca ggagccgccc tgcagatccc ctttgccatg 2700

cagatggcct atcggttcaa cggcatcggc gtgacccaga atgtgctgta cgagaaccag 2760

aagctgatcg ccaatcagtt taacagcgcc atcggcaaga tccaggactc tctgagctcc 2820

accgccagcg ccctgggcaa gctgcaggat gtggtgaatc agaacgccca ggccctgaat 2880

acactggtga agcagctgtc tagcaacttc ggcgccatct cctctgtgct gaatgacatc 2940

ctgagccggc tggacaaggt ggaggcagag gtgcagatcg accggctgat caccggcaga 3000

ctgcagtccc tgcagaccta cgtgacacag cagctgatca gggcagcaga gatcagggcc 3060

tctgccaatc tggccgccac aaagatgagc gagtgcgtgc tgggacagtc caagagggtg 3120

gacttttgcg gcaagggcta tcacctgatg agcttcccac agtccgcccc tcacggagtg 3180

gtgtttctgc acgtgaccta cgtgccagcc caggagaaga acttcaccac agcccccgcc 3240

atctgtcacg atggcaaggc ccactttcct agggagggcg tgttcgtgag caacggcacc 3300

cactggtttg tgacacagcg caatttctac gagccacaga tcatcaccac agacaatacc 3360

ttcgtgtccg gcaactgcga cgtggtcatc ggcatcgtga acaatacagt gtatgatcct 3420

ctgcagccag agctggactc ttttaaggag gagctggata agtacttcaa gaatcacacc 3480

agccccgacg tggatctggg cgacatctct ggcatcaatg ccagcgtggt gaacatccag 3540

aaggagatcg acagactgaa cgaggtggcc aagaatctga acgagagcct gatcgatctg 3600

caggagctgg gcaagtatga gcagtacatc aagtggccct ggtatatctg gctgggcttc 3660

atcgccggcc tgatcgccat cgtgatggtg accatcatgc tgtgctgtat gacaagctgc 3720

tgttcctgcc tgaagggctg ctgttcttgt ggcagctgct gtaagtttga tgaggacgat 3780

tccgagcctg tgctgaaggg cgtgaagctg cactacacct aa 3822

<210> 2
<211> 59
<212> DNA
<213> Artificial Sequence

<400> 2
gcgtttaaac ttaagcttgg taccgagctc ggatccgcca ccatgtttgt ttttcttgt 59

<210> 3
<211> 42
<212> DNA
<213> Artificial Sequence

<400> 3
agaatagggc cctctagact agtttatgtg taatgtaatt tg 42

<210> 4
<211> 59
<212> DNA
<213> Artificial Sequence

<400> 4
gcgtttaaac ttaagcttgg taccgagctc ggatccgcca ccatgttcgt gtttctggt 59

<210> 5
<211> 45
<212> DNA
<213> Artificial Sequence

<400> 5
agaatagggc cctctagact agtttatcag gtgtagtgca gcttc 45

<210> 6
<211> 26
<212> DNA
<213> Artificial Sequence

<400> 6

tctagagggc cctattctat agtgtc 26

<210> 7
<211> 46
<212> DNA
<213> Artificial Sequence

<400> 7
ggatccgagc tcggtaccaa gcttaagttt aaacgctaga gtccgg 46

## Description

### TECHNICAL FIELD

The present invention relates to an Ad7-vectored vaccine for preventing SARS-CoV-2 infection.

### BACKGROUND ART

Vaccines are the most economical and effective intervention measures to prevent and control SARS-CoV-2 infection. The published results obtained from the alignment of more than 100 SARS-CoV-2 virus genomes have shown that the overall mutation degree of the virus is relatively low, and no recombination phenomenon has been found. Therefore, SARS-CoV-2 vaccines, if successfully developed, will protect human from SARS-CoV-2 infection, thereby preventing the outbreak of a new epidemic.

In the virus particle structure of the SARS-CoV-2 coronavirus, S protein which constitutes the "crown" is an obvious target, and has become the research focus of most research teams. Through computer simulation of the three-dimensional structure of S protein, some research teams have successfully revealed the relationship between the S protein and an ACE2 receptor during the invasion of cells . Therefore, the S protein plays an important role in mediating the binding of a virus particle to a host cell receptor and inducing a neutralizing antibody.

The complete sequence of SARS-CoV-2 is as shown in NC_045512.2, in which the 21563..25384 nucleic acid is a coding sequence of Spike protein (S). The S protein is a major structural protein of a virus particle, and plays an important role in mediating the binding of a virus particle to a host cell receptor and inducing a neutralizing antibody. Therefore, for vaccines with S protein as an antigen, including nucleic acid vaccines, subunit vaccines and virus-vectored vaccines, the expression levels and protein structures of S proteins determine the efficacies of such vaccines.

However, experiments have shown that the expression level of the gene of Spike protein (S) of natural SARS-CoV-2 in a human kidney cells HEK293 is very low. Therefore, if the original S codons are used for expression as the antigen, the vaccine may be ineffective or have a low titer, which is not enough to resist the virus infection.

Adenoviruses are common vectors in vaccine research and gene therapies, and have been widely used in the biomedical field. Compared with other virus vectors, the adenoviruses have a low toxicity, and infection with adenoviruses only cause mild cold symptoms. At present, most adenovirus-vectored vaccines developed by research teams are based on adenovirus type 5. Preformed antibodies against adenovirus type 5 vectors have a very high proportion in the human population, which will affect the titer of an adenovirus type 5 vectored vaccines to a certain extent.

### SUMMARY OF THE INVENTION

An object of the present disclosure is to provide an Ad7-vectored vaccine for preventing SARS-CoV-2 infection, in order to overcome at least one deficiency of the prior art.

The present disclosure blocks SARS-CoV-2 infection by integrating an optimized S gene onto an Ad7 vector, especially a replication-defective Ad7 vector, infecting cells via the adenovirus, presenting an novel coronavirus antigen, and producing a specific immune response in an organism after immunization.

The technical solutions used in the present disclosure are as follows:
An adenovirus vectored vaccine for preventing SARS-CoV-2 infection, comprising an Ad7 vector loaded with a nucleic acid sequence as shown in SEQ ID NO: 1.

To further improve the safety of the vaccine, in some embodiments, the Ad7 vector is a replication-defective Ad7 vector.

In some embodiments, the replication-defective Ad7 vector is a replication-defective Ad7 vector with genes in E1 and E3 regions deleted.

In some embodiments, the nucleotide sequence can be expressed as protein in a human-derived cell or the human body.

In some embodiments, in the human body, the protein is capable of:
inducing an immune response; or
generating a biology reporter molecule; or
generating a trace molecule for detection; or
regulating gene function; or
functioning as a therapeutic molecule.

In some embodiments, the adenovirus vectored vaccine further comprises a pharmaceutically acceptable adjuvant, vector, diluent or excipient. At least one from the group consisting of suitable adjuvant, vector, diluent or excipient may be selected according to specific needs.

In some embodiments, the adenovirus vectored vaccine further comprises at least one medicament useful for treating COVID-19.

In some embodiments, the dosage form of the adenovirus vectored vaccine includes, but not limited to common vaccine dosage forms, such as an oral preparation, an injection, an aerosol inhalant.

In some embodiments, the adenovirus vectored vaccine may also be used in combination with other vaccines.

To further improve the safety of products, in some embodiments, the Ad7 vector is capable of regulating the expression of the nucleic acid sequence as shown in SEQ ID NO: 1.

In some embodiments, the transcription direction of the nucleic acid sequence shown in SEQ ID NO: 1 is opposite to those of the other genes in the Ad7 vector. In this way, the nucleic acid sequence shown in SEQ ID NO: 1 can be better expressed.

The beneficial effects of the present disclosure are as follows:

Some embodiments of the disclosure have good safety and are convenient to use. Experiments have shown that the vaccine is capable of producing more S protein in human cells, which is expected to be developed as an Ad7-vectored vaccine for preventing SARS-CoV-2 infection.

Some embodiments of the present disclosure may be used in combination with other vaccines, and may also be used as therapeutic vaccines for COVID-19. When a patient is vaccinated with the vaccine of the present disclosure at the initial stage of infection, the vaccine can quickly induce an immune response in the human body, thereby achieving a therapeutic effect.

### BRIEF DESCRIPTION OF THE DRAWINGS

FIG. 1 shows detection results of the expression of S protein.
FIG. 2 shows detection results of binding antibodies in serum of mice 28 days after immunization.
FIG. 3 shows detection results of binding antibodies in serum of macaque monkeys at different times after immunization.
FIG. 4 shows ELISpot detection results of peripheral blood PBMCs of the macaque monkeys 18 days after immunization.

### DETAILED DESCRIPTION

The amino acid sequence of Spike (S) protein of SARS-CoV-2 is shown in YP_009724390.1, which is denoted as NB1.

The pre-mRNA transcribed by eukaryotic cells can produce various mRNA splicing isoforms by various splicing modes (by selecting different splicing site combinations), which ultimately leads to various proteins resulting from the same gene sequence. This is very unfavorable for the expression of the protein. By performing codon optimization on the wild-type natural nucleic acid sequence and removing potential variable splicing sites based on self-owned technology, the inventors ensured the uniqueness of the expression of the protein and reduced the difficulty in the subsequent purification of the protein. The optimized nucleic acid sequence is denoted as NB2, and the specific sequence is shown in SEQ ID NO: 1:

ATGTTCGTGTTTCTGGTGCTGCTGCCTCTGGTGAGCTCCCAGTGCGTGAACCTGACCAC
AAGGACCCAGCTGCCACCTGCCTATACCAATAGCTTCACACGGGGCGTGTACTATCCCG
ACAAGGTGTTTAGATCTAGCGTGCTGCACTCCACCCAGGATCTGTTTCTGCCTTTCTTTT

CTAACGTGACATGGTTCCACGCCATCCACGTGTCCGGCACCAATGGCACAAAGCGGTTC
GACAATCCAGTGCTGCCCTTTAACGATGGCGTGTACTTCGCCTCCACCGAGAAGTCTAA
CATCATCAGAGGCTGGATCTTTGGCACCACACTGGACAGCAAGACCCAGTCCCTGCTGA
TCGTGAACAATGCCACAAACGTGGTCATCAAGGTGTGCGAGTTCCAGTTTTGTAATGAT
CCCTTCCTGGGCGTGTACTATCACAAGAACAATAAGTCTTGGATGGAGAGCGAGTTTAG
GGTGTATTCCTCTGCCAACAATTGCACCTTTGAGTACGTGAGCCAGCCTTTCCTGATGGA
CCTGGAGGGCAAGCAGGGCAATTTCAAGAACCTGAGGGAGTTCGTGTTTAAGAATATCG
ATGGCTACTTCAAGATCTACTCCAAGCACACACCAATCAACCTGGTGCGCGACCTGCCA
CAGGGCTTCTCTGCCCTGGAGCCACTGGTGGATCTGCCCATCGGCATCAACATCACCCG
GTTTCAGACACTGCTGGCCCTGCACAGAAGCTACCTGACCCCAGGCGACAGCTCCTCTG
GATGGACAGCAGGAGCTGCCGCCTACTATGTGGGCTATCTGCAGCCCCGCACCTTCCTG
CTGAAGTACAACGAGAATGGCACCATCACAGACGCAGTGGATTGCGCCCTGGACCCCCT
GTCTGAGACCAAGTGTACACTGAAGAGCTTTACAGTGGAGAAGGGCATCTACCAGACC
AGCAACTTCAGGGTGCAGCCAACAGAGTCCATCGTGCGCTTTCCCAATATCACCAACCT
GTGCCCTTTTGGCGAGGTGTTCAATGCCACACGCTTCGCCAGCGTGTACGCCTGGAATA
GGAAGCGCATCTCCAACTGCGTGGCCGACTATTCTGTGCTGTACAACAGCGCCTCCTTC
TCTACCTTTAAGTGTTATGGCGTGAGCCCCACCAAGCTGAATGATCTGTGCTTTACAAAC
GTGTACGCCGATTCCTTCGTGATCAGGGGCGACGAGGTGCGCCAGATCGCACCAGGACA
GACCGGCAAGATCGCAGACTACAATTATAAGCTGCCTGACGATTTCACAGGCTGCGTGA
TCGCCTGGAACTCTAACAATCTGGATAGCAAAGTGGGCGGCAACTACAATTATCTGTACC
GGCTGTTTAGAAAGTCTAATCTGAAGCCATTCGAGCGGGACATCTCCACCGAGATCTAC
CAGGCCGGCTCTACACCCTGCAATGGCGTGGAGGGCTTTAACTGTTATTTCCCTCTGCAG
TCCTACGGCTTCCAGCCAACCAACGGCGTGGGCTATCAGCCCTACAGAGTGGTGGTGCT
GTCTTTTGAGCTGCTGCACGCACCTGCAACCGTGTGCGGCCCAAAGAAGAGCACAAAT
CTGGTGAAGAACAAGTGCGTGAACTTCAACTTCAACGGACTGACCGGCACAGGCGTGC
TGACCGAGAGCAACAAGAAGTTCCTGCCATTTCAGCAGTTCGGCAGGGACATCGCAGA
TACCACAGACGCCGTGCGCGACCCTCAGACCCTGGAGATCCTGGACATCACACCATGTT
CCTTCGGCGGCGTGTCTGTGATCACCCCAGGCACCAATACATCCAACCAGGTGGCCGTG
CTGTATCAGGACGTGAATTGCACAGAGGTGCCCGTGGCAATCCACGCAGATCAGCTGAC
CCCTACATGGCGGGTGTACTCTACCGGCAGCAACGTGTTCCAGACAAGAGCCGGATGCC
TGATCGGAGCAGAGCACGTGAACAATAGCTATGAGTGCGACATCCCTATCGGCGCCGGC
ATCTGTGCCTCCTACCAGACCCAGACAAACTCCCCAAGGAGAGCCCGGTCTGTGGCCA
GCCAGTCCATCATCGCCTATACCATGAGCCTGGGCGCCGAGAACAGCGTGGCCTACTCC
AACAATTCTATCGCCATCCCTACCAACTTCACAATCAGCGTGACCACAGAGATCCTGCCA
GTGAGCATGACCAAGACATCCGTGGACTGCACCATGTATATCTGTGGCGATTCCACAGA

GTGTTCTAACCTGCTGCTGCAGTACGGCTCCTTTTGCACCCAGCTGAATAGAGCCCTGAC
AGGCATCGCCGTGGAGCAGGACAAGAACACCCAGGAGGTGTTCGCCCAGGTGAAGCA
GATCTACAAGACACCACCCATCAAGGACTTTGGCGGCTTCAACTTCAGCCAGATCCTGC
CCGATCCTAGCAAGCCATCCAAGCGGTCTTTTATCGAGGACCTGCTGTTCAACAAGGTG
ACCCTGGCCGATGCCGGCTTCATCAAGCAGTATGGCGATTGTCTGGGCGACATCGCCGC
CAGAGACCTGATCTGCGCCCAGAAGTTTAATGGCCTGACCGTGCTGCCTCCACTGCTGA
CAGATGAGATGATCGCACAGTACACCTCTGCCCTGCTGGCCGGCACCATCACAAGCGGA
TGGACATTCGGCGCAGGAGCCGCCCTGCAGATCCCCTTTGCCATGCAGATGGCCTATCG
GTTCAACGGCATCGGCGTGACCCAGAATGTGCTGTACGAGAACCAGAAGCTGATCGCC
AATCAGTTTAACAGCGCCATCGGCAAGATCCAGGACTCTCTGAGCTCCACCGCCAGCGC
CCTGGGCAAGCTGCAGGATGTGGTGAATCAGAACGCCCAGGCCCTGAATACACTGGTG
AAGCAGCTGTCTAGCAACTTCGGCGCCATCTCCTCTGTGCTGAATGACATCCTGAGCCG
GCTGGACAAGGTGGAGGCAGAGGTGCAGATCGACCGGCTGATCACCGGCAGACTGCAG
TCCCTGCAGACCTACGTGACACAGCAGCTGATCAGGGCAGCAGAGATCAGGGCCTCTG
CCAATCTGGCCGCCACAAAGATGAGCGAGTGCGTGCTGGGACAGTCCAAGAGGGTGGA
CTTTTGCGGCAAGGGCTATCACCTGATGAGCTTCCCACAGTCCGCCCCTCACGGAGTGG
TGTTTCTGCACGTGACCTACGTGCCAGCCCAGGAGAAGAACTTCACCACAGCCCCCGCC
ATCTGTCACGATGGCAAGGCCCACTTTCCTAGGGAGGGCGTGTTCGTGAGCAACGGCAC
CCACTGGTTTGTGACACAGCGCAATTTCTACGAGCCACAGATCATCACCACAGACAATA
CCTTCGTGTCCGGCAACTGCGACGTGGTCATCGGCATCGTGAACAATACAGTGTATGATC
CTCTGCAGCCAGAGCTGGACTCTTTTAAGGAGGAGCTGGATAAGTACTTCAAGAATCAC
ACCAGCCCCGACGTGGATCTGGGCGACATCTCTGGCATCAATGCCAGCGTGGTGAACAT
CCAGAAGGAGATCGACAGACTGAACGAGGTGGCCAAGAATCTGAACGAGAGCCTGATC
GATCTGCAGGAGCTGGGCAAGTATGAGCAGTACATCAAGTGGCCCTGGTATATCTGGCT
GGGCTTCATCGCCGGCCTGATCGCCATCGTGATGGTGACCATCATGCTGTGCTGTATGAC
AAGCTGCTGTTCCTGCCTGAAGGGCTGCTGTTCTTGTGGCAGCTGCTGTAAGTTTGATG
AGGACGATTCCGAGCCTGTGCTGAAGGGCGTGAAGCTGCACTACACCTAA (SEQ ID NO.:
1).

### Construction of Ad7 vector for S protein expression (pAd7-NB2):

Using NB 1 and NB2 as templates respectively, an NB1 fragment was obtained through PCR amplification with primers NB1-F and NB1-R, and an NB2 fragment was obtained through PCR amplification with primers NB2-F and NB2-R. A vector plasmid backbone pGK was obtained through PCR amplification by using CMV-R and BGH-F as primers and using pGA1-EGFP plasmid as a template. Then, *in vitro* two-fragment recombination with the NB1 and NB2 fragments was carried out, respectively, by using a homologous recombination enzyme (Exnase), to obtain pGK-NB1 and pGK-NB2. The pGK-NB2 was linearized by using Bstz17I and SgarAI, and was subjected to homologous recombination with pAd7ΔE1ΔE3 (i.e., Ad7 empty vector), which was digested to delete the unique restriction site in the E1 region and linearized, by using competent BJ5183 to construct a pAd7-NB2 vaccine vector.

### Primer sequences for amplifying NB1:

### NB1-F:

GCGTTTAAACTTAAGCTTGGTACCGAGCTCGGATCCGCCACCATGTTTGTTTTTCTTGT
(SEQ ID NO.: 2)

NB1-R: AGAATAGGGCCCTCTAGACTAGTTTATGTGTAATGTAATTTG (SEQ ID NO.: 3)

Primer sequences for amplifying NB2:

### NB2-F:

GCGTTTAAACTTAAGCTTGGTACCGAGCTCGGATCCGCCACCATGTTCGTGTTTCTGGT
(SEQ ID NO.: 4)

NB2-R: AGAATAGGGCCCTCTAGACTAGTTTATCAGGTGTAGTGCAGCTTC (SEQ ID NO.: 5)

Primer sequences for amplifying pGK:

BGH-F: TCTAGAGGGCCCTATTCTATAGTGTC (SEQ ID NO.: 6)

CMV-R: GGATCCGAGCTCGGTACCAAGCTTAAGTTTAAACGCTAGAGTCCGG (SEQ ID
NO.: 7)

PCR conditions: 95°C for 3 min; 95°C for 30 s; 60°C for 30 s; 72°C for 2 min; 30 cycles; and 72°C for 5 min.

Rescue and production of Ad7-NB2 vector:
1) according to a conventional method, pAd7-NB2 was linearized with AsiSI, recovered by precipitation in ethanol, and transfected into 293 cells by means of cationic lipofection;
2) 8 hours after the transfection, 2 ml of DMEM medium containing 5% fetal bovine serum was added, incubated for 7-10 days, and cytopathic effect observation was carried out;
3) after the cytopathic effect was observed, the cells and culture supernatant were collected, freeze-thawed for three times in a water bath at 37°C and liquid nitrogen, and centrifugated to remove cell debris, and then a 10 cm-dish was infected with the supernatant;
4) after 2 or 3 days, the cells and culture supernatant were collected, freeze-thawed for 3 times, centrifugated to remove cell debris, and then 3-5 15 cm-dishes were infected with the supernatant;
5) after 2 or 3 days, the cells were collected, freeze-thawed for 3 times, and centrifugated to remove cell debris;
6) 30 15 cm-dishes were infected with the supernatant, after 2 or 3 days, the cells were collected, freeze-thawed for 3 times, and centrifugated to remove cell debris;
7) the supernatant was added to a cesium chloride density gradient centrifuge tube;, and centrifuged at 4°C, 40000 rpm, for 4 hours, then a virus band was pipetted out, desalted, and subpackaged; and
8) the virion titer was determined by means of OD260 absorbance, with the calculation formula being: virus concentration = OD260 × dilution multiple × 36/genome length (Kb), and the virus stock solution was cryopreserved at -80°C.

### Detection of Spike gene expression:

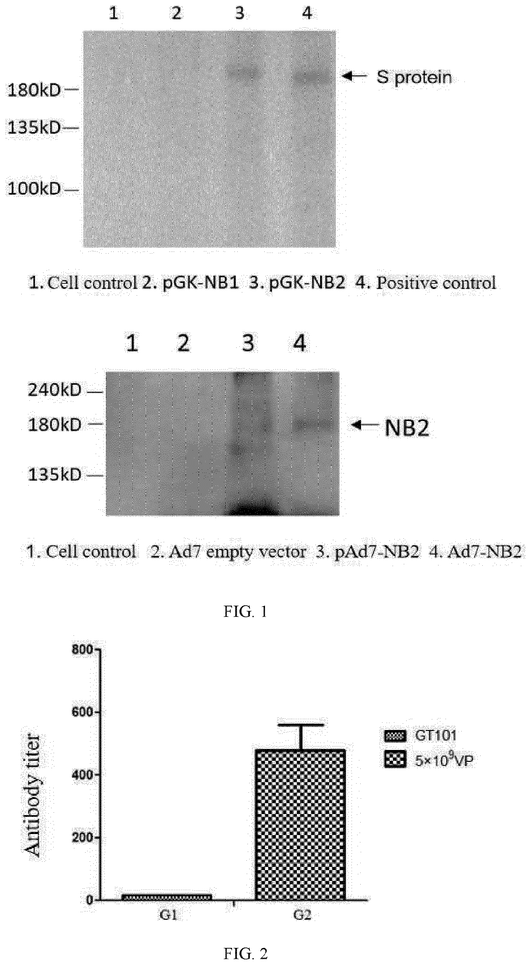

According to a conventional method, 2.5 µg of pGK-NB1 and pGK-NB2 were respectively transfected using cationic liposomes, and after 48 hours, the cells were collected. The HEK293 cells were infected with Ad7-NB2 and Ad7 empty vector viruses, and after 24 hours, the cells were collected. The four samples mentioned above were processed according to the conventional Western Blot method, and were detected for the protein (FIG. 1).

It could be seen from FIG. 1 that no S protein expression was detected in the pGK-NB1 sample, while the expression of S protein could be observed in the codon-optimized pGK-NB2 and vaccine candidate strain Ad7-NB2 samples, indicating that the NB2 sequence has unexpected effects.

Immunogenicity evaluation of Ad7-NB2 vaccine:

### 1. Immunized mice

An immunogenicity evaluation scheme of the Ad7-NB2 vaccine in mice was designed, as shown in Table 1, and immunization was carried out according to the designed immunization scheme.

**Table 1**

| **Grouping** | **Primary immunization (0 day)** | | | **Detection (28^{th} day)** |
|---|---|---|---|---|
| | Vaccine type | Vaccine dosage | | |
| G1 | GT101 | - | 100ul | Detection of binding antibody against S protein. |
| G2 | Ad7-NB2 | 5×10⁹VP/animal | 1 ml | |

Balb/c mice, 6 to 8 weeks old, were divided into 2 groups with 5 mice in each group. The G1 group was an experimental control group and mice in the G1 group were intramuscularly injected with GT101 (the virus stock solution) at a dosage of 5×10⁹VP/animal, while the G2 group was an experimental group and mice in the G2 group were intramuscularly injected with the Ad7-NB2 vaccine at a dosage of 5×10⁹VP/animal. At the 28^{th} day after immunization, blood was taken from orbit, and serum was separated. Binding antibody levels of the S protein of SARS-ncov2 were detected. From FIG. 2, the experimental group could produce a high titer of binding antibody against the S protein of SARS-ncov2 after immunization.

### 2. Immunized macaque monkeys

The macaque monkeys were obtained from Guangdong Landau Biotechnology Co. Ltd. The vaccinated macaque monkeys were 2 to 3 years old. The macaque monkeys were randomly divided into 3 groups, with 2 monkeys in each experimental group, and 4 monkeys in each control group, specifically as shown in Table 2:

**Table 2**

| Groups No. | Macaque monkeys | Gender | Immunization dose/mode |
|---|---|---|---|
| 1 | 170082 | Female | Ad7-NB2(1×10¹¹ vp) Intramuscular (I.M.) injection |
| | 170018 | Female | |
| 2 | 181045 | Male | Ad7-NB2(1×10¹¹ vp) Intranasal (I.N.) immunization |
| | 180021 | Male | |
| 3 | 180026 | Female | — |
| | 180024 | Female | |
| | 180023 | Male | |
| | 180039 | Male | |

Macaque monkeys were immunized with the vaccine prepared from the Ad7-NB2 virus strain. Blood was taken at the 14^{th} day and the 18^{th} day after immunization to determine antibody binding titers by ELISA. The peripheral blood was separated and detected for a cellular immune response by ELISpot assay.

### Experimental results:

### (1) Binding antibodies

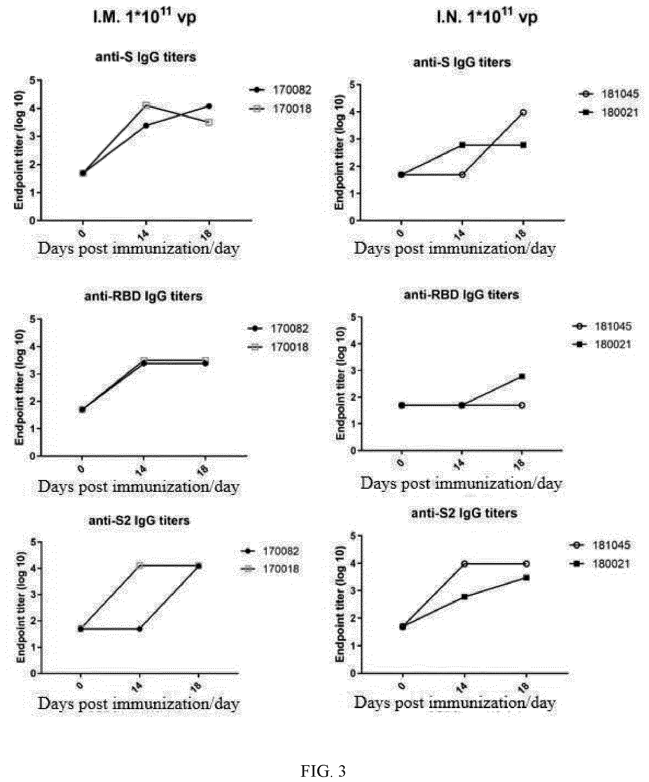

At the 14^{th} day and the 18^{th} day after intramuscular vaccination, significant presence of S-specific IgG and RBD-specific IgG could be detected in the sera of all the macaque monkeys intramuscularly injected with 1×10¹¹ VP, and among the two macaque monkeys in the immunization group, significant presence of anti-S2 IgG could be detected in one macaque monkey (FIG. 3).

At the 14^{th} day after the intranasal vaccination, among the two macaque monkeys vaccinated with 1×10¹¹ VP in the immunization group, significant presence of S-specific IgG and RBD-specific IgG could be detected in one macaque monkey. S2-specific IgG could be detected in all macaque monkeys in the immunization group. At the 18^{th} day after immunization, S-specific IgG and S2-specific IgG could both be detected. Among the two macaque monkeys in the immunization group, significant presence of RBD-specific IgG could be detected in one monkey (FIG. 3).

The difference between the antibody titers induced by intramuscular injection immunization and intranasal immunization was small.

### (2) Cellular Immunity

To determine whether Ad7-NB2 could also induce a cellular immune response in non-human primates (NHPs), the responses of S-specific IFN-γ secreting cells from the peripheral blood mononuclear cells (PBMCs) to S1 and S2 peptide libraries were detected.

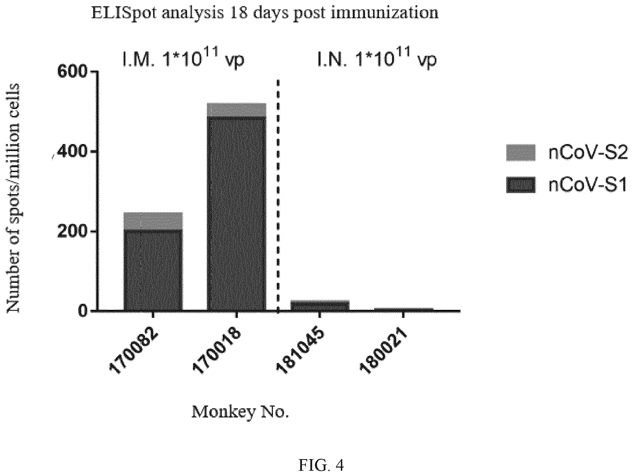

The results showed that:
1) At the 18^{th} day after the intramuscular injection vaccination, all macaque monkeys intramuscularly injected with 1×10¹¹VP had cellular immune responses to the S1 and S2 peptide libraries (FIG. 4).
2) At the 18^{th} day after the intranasal vaccination, among the two vaccinated macaque monkeys, one macaque monkey showed weak cellular immune response to the S1 peptide library, and neither of them had obvious response to the S2 peptide library at the 18^{th} day (FIG. 4).

Therefore, in the macaque monkeys, the cellular immune response was mainly directed at the S1 region. These results indicate that the intramuscular injection immunization of the vaccine could cause systemic cellular immune response to S protein, especially to the S1 region, while the systemic cellular immune response caused by the mucosal immunization of the vaccine was relatively weak.

The above experimental results showed that the vaccine could induce cellular immunity in macaque monkeys, which may further enhance the protective effect on the body.

## Claims

1. An adenovirus vectored vaccine for preventing SARS-CoV-2 infection, comprising an Ad7 vector loaded with a nucleic acid sequence shown in SEQ ID NO: 1.

2. The adenovirus vectored vaccine of claim 1, wherein the Ad7 vector is a replication-defective Ad7 vector.

3. The adenovirus vectored vaccine of claim 2, wherein the replication-defective Ad7 vector is a replication-defective Ad7 vector with genes in E1 and E3 regions deleted .

4. The adenovirus vectored vaccine of any one of claims 1 to 3, wherein the nucleotide sequence can be expressed as protein in a human-derived cell or the human body.

5. The adenovirus vectored vaccine of claim 4, wherein, in the human body, the protein is capable of:
inducing an immune response; or
generating a biology reporter molecule; or
generating a trace molecule for detection; or
regulating gene function; or
functioning as a therapeutic molecule.

6. The adenovirus vectored vaccine of any one of claims 1 to 3, further comprising a pharmaceutically acceptable adjuvant, vector, diluent or excipient.

7. The adenovirus vectored vaccine of any one of claims 1 to 3, further comprising at least one medicament useful for treating COVID-19.

8. The adenovirus vectored vaccine of any one of claims 1 to 3, wherein the Ad7 vector is capable of regulating the expression of the nucleic acid sequence shown in SEQ ID NO: 1.

9. The adenovirus vectored vaccine of any one of claims 1 to 3, wherein the transcription direction of the nucleic acid sequence shown in SEQ ID NO: 1 is opposite to those of the other genes in the Ad7 vector.
